(19) Europäisches Patentamt
European Patent Office
Office européen des brevets

(11) Veröffentlichungsnummer: **0 615 439 B1**

(12) # EUROPÄISCHE PATENTSCHRIFT

(45) Veröffentlichungstag der Patentschrift: **08.11.95**

(51) Int. Cl.[6]: **A61K 9/10**, A61K 9/51, A61K 9/20, A61K 31/19

(21) Anmeldenummer: **92924545.4**

(22) Anmeldetag: **04.12.92**

(86) Internationale Anmeldenummer: **PCT/DE92/01007**

(87) Internationale Veröffentlichungsnummer: **WO 93/10760 (10.06.93 93/14)**

(54) **EIN IBUPROFEN ENTHALTENDES RETARD-ARZNEIMITTEL UND SEINE VERWENDUNG.**

(30) Priorität: **05.12.91 DE 4140172**

(43) Veröffentlichungstag der Anmeldung: **21.09.94 Patentblatt 94/38**

(45) Bekanntmachung des Hinweises auf die Patenterteilung: **08.11.95 Patentblatt 95/45**

(84) Benannte Vertragsstaaten: **AT BE CH DE DK ES FR GB GR IT LI NL PT SE**

(56) Entgegenhaltungen:
**EP-A- 0 267 321**
**EP-A- 0 275 796**
**EP-A- 0 282 020**
**EP-A- 0 290 168**
**WO-A-90/14081**
**WO-A-91/06292**
**FR-A- 2 608 427**

**CHEMICAL ABSTRACTS, vol. 115, no. 18, 4. November 1991, Columbus, Ohio, US; abstract no. 189667e, BODMEIER R. ET AL 'SPONTANEOUS FORMATION OF DRUG-CONTAINING ACRYLIC NANOPARTICLES' Seite 420 ;Spalte 2 ;**

(73) Patentinhaber: **ALFATEC-PHARMA GmbH**
**Im Neuenheimer Feld 519**
**D-69120 Heidelberg (DE)**

(72) Erfinder: **WUNDERLICH, Jens-Christian**
**Bothestrasse 52**
**D-6900 Heidelberg (DE)**
Erfinder: **SCHUSTER, Otto**
**Kelkheimerstrasse 69**
**D-6232 Bad Soden (DE)**
Erfinder: **LUKAS, Helmut**
**Taunusstrasse 30**
**D-6078 Neu-Isenburg (DE)**
Erfinder: **SCHICK, Ursula**
**Staatsbahnhofstrasse 6**
**D-6908 Wiesloch (DE)**

(74) Vertreter: **Fürniss, Peter, Dr.**
**Kuhnen, Wacker & Partner**
**Alois-Steinecker-Strasse 22**
**D-85354 Freising (DE)**

**EP 0 615 439 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Retard-Arzneimittel zur Behandlung von schmerzhaften und/oder entzündlichen sowie fieberhaften Erkrankungen, das Ibuprofen neben üblichen pharmazeutischen Trägern und Hilfsstoffen enthält, das dadurch gekennzeichnet ist, daß das Ibuprofen in Form eines pharmazeutisch applizierbaren Nanosols vorliegt, das als Träger im wesentlichen Gelatine, fraktionierte Gelatine oder ein Gelatinederivat enthält.

Weiterhin betrifft die Erfindung die Verwendung eines pharmazeutisch applizierbaren Nanosols von Ibuprofen zur Herstellung von Arzneimitteln mit analgetischer und/oder antirheumatischer Retardwirkung.

Die Erfindung betrifft schließlich ein solches Arzneimittel und seine Verwendung, bei dem das Ibuprofen oder seine Enantiomeren teilweise als Akutform, teilweise als Retardform vorliegt.

Ibuprofen (2-(4-Isobutylphenyl)propionsäure $C_{13}H_{18}O_2$) der folgenden Struktur

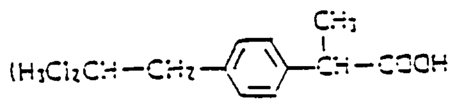

ist ein bekanntes Schmerz- und Rheumamittel, das sich über die Prostaglandinsythesehemmung beispielsweise in tierexperimentellen Entzündungsmodellen als wirksam erwiesen hat. In der Humantherapie reduziert Ibuprofen entzündlich bedingte Schmerzen, Schwellungen und Fieber. Der empfohlene Dosierungsbereich für die perorale Applikation liegt bei maximalen Einzeldosen von 800 mg zwischen 1,2 und 2,4 g Ibuprofen pro Tag und ist damit im Vergleich zu anderen NSAR verhältnismäßig hoch. Einzeldosen sollten 200 mg nicht unterschreiten und in der Regel bei 400 bis 800 mg liegen, um ausreichende antiphlogistische Effekte bei rheumatischen Erkrankungen zu erzielen. Es ist jedoch auch bekannt, daß das Racemat vereinzelt schwere, unerwünschte Nebenwirkungen, wie z.B. Beeinträchtigungen des Magen-Darm-Traktes, Ausbildung von Magenulcera, Diarrhoen oder zentrale Nebenwirkungen erzeugen kann.

Normalerweise fällt Ibuprofen bei der chemischen Synthese als Racemat an. Entgegen der etablierten Meinung, daß Ibuprofen-Racemat die ideale therapeutisch wirksame Form darstellt, zeigten Untersuchungen in jüngerer Zeit, daß reines S-Ibuprofen gegenüber dem Racemat ein wesentlich größeres pharmakologisches Potential besitzt. Es wurde gefunden, daß bei alleiniger Verwendung von S-Ibuprofen als Wirkstoff, die Dosis nicht nur halbiert werden konnte, sondern sogar mit weniger als der halben Dosis der S-Form im Vergleich zum Racemat gleiche therapeutische Wirkung erzielt werden konnte. Die Herstellung der enantiomerreinen Substanzen S- und R-Ibuprofen wird durch neue Verfahren der Diastereomerentrennung jetzt auch großtechnisch möglich (EP 0362 476). Diese enantiomerreinen Stoffe stellen einen zukunftsweisenden therapeutischen Fortschritt bei der Behandlung von Schmerzen und Erkrankungen des rheumatischen Formenkreises dar.

In der deutschen Patentanmeldung P 4028906.0 der PAZ-Arzneimittelentwicklungsgesellschaft mbH wird beschrieben, daß sich eine synthetische Mischung (Pseudoracemat) des 2-Arylpropionsäurederivates Flurbiprofen, die aus je 50% des S- und des R-Enantiomeren zusammengesetzt wurde, bezüglich ihrer Auflösungsgeschwindigkeit (Freisetzung) im wäßrigem Milieu anders verhält als das aus je 50% S- und R-Enantiomeren bestehende, bei der Synthese anfallende racemische Flurbiprofen. Tatsächlich ist die Auflösungsgeschwindigkeit des Pseudoracemats wesentlich höher als die des Racemats, wodurch sich auch wesentlich höhere Blutspiegel erreichen lassen. Dieser Effekt ist bei Ibuprofen noch stärker ausgeprägt, da sich die racemische Verbindung und die Enantiomeren bzw. Enantiomermischungen in der Kristallform, in der Löslichkeit, in der Lösungsgeschwindigkeit und im Schmelzpunkt noch deutlicher unterscheiden, als dies bei Flurbiprofen der Fall ist. So beträgt die Löslichkeit des Racemats bei 37 °C 4,8 mg/100 ml Wasser, der Schmelzpunkt ist 75-77,5 °C. S-Ibuprofen dagegen zeigt unter gleichen Bedingungen eine Löslichkeit von 11,8 mg/100 ml Wasser und einen Schmelzpunkt von 51-52 °C.

Trotz vielfältiger galenischer Entwicklungen peroraler Retardzubereitungen ist es für Ibuprofen, seine Enantiomeren und deren Mischungen mit unterschiedlichen Anteilen an S- und R-Ibuprofen bis heute noch nicht gelungen, den in-vivo Resorptionsprozeß des aus einer entsprechenden Arzneiform primär freigegebenen Wirkstoffs so optimal an die physiologischen Gegebenheiten (pH-Verhältnisse im Gastrointestinaltrakt, gastrointestinale Verweilzeiten von Formlingen, spezifische Resorptionsfenster für bestimmte Wirkstoffe) anzupassen, daß die Hauptanforderungen an eine Retardarzneiform erfüllt werden.

Retardierung eines Wirkstoffs in einer pharmazeutischen Darreichungsform ist dann erwünscht, wenn die biologische Halbwertszeit dieses Wirkstoffs kurz ist (in der Regel ca. 2h), wobei sich Ibuprofen mit einer $t_{\frac{1}{2}}$ von 2,4 h besonders gut eignet. Durch lang anhaltende Freigabe aus der Arzneiform im Organismus

erhofft man sich verschiedene Vorteile:

1.) Verbesserte Wirkung
- Möglichst genaue Einstellung von Plasmaspiegeln im therapeutischen Niveau soll einerseits Plasmaspiegelschwankungen vermindern, andererseits Nebenwirkungen (u.U. toxisch) vermeiden.

2.) Verlängerte Wirkung
- damit verbunden ist analog eine entsprechende Reduktion der Einnahmehäufigkeit und dadurch eine entscheidende Erhöhung der Patienten-Compliance.

3.) Verminderung der insgesamt verabreichten Arzneistoffdosis bei Erzielung vergleichbarer Wirkung gegenüber der mehrfachen Einzelgabe.

Die galenische Konzeption von bekannten Retardarzneiformen ist i.a. so angelegt, daß die Wirkstofffreigabe im Organismus den geschwindigkeitsbestimmenden Schritt im Freigabe-Resorptions-Geschehen darstellt. Aus einem Depot soll der Wirkstoff verzögert und möglichst gleichmäßig (idealerweise konstant = Kinetik nullter Ordnung) freigegeben werden, um einen konstanten Übergang in die Biophase zu erreichen. Es ist jedoch bisher noch so, daß besagter Wirkstoff nach seiner Freisetzung weitestgehend unkontrollierbar seinem Schicksal überlassen bleibt, d.h. physiologische Einflußgrößen im Gastrointestinaltrakt bleiben unberücksichtigt.

Dies trifft besonders für die schwer wasserlösliche, schwache Wirkstoffsäure Ibuprofen ($pK_A$ = 4,6) zu, die daher zu den Wirkstoffen mit problematischer Bioverfügbarkeit gezählt werden muß. Trotz einer Vielzahl von verschiedenen galenischen Entwicklungen scheint es noch nicht gelungen zu sein, eine Formulierung für Ibuprofen zu entwickeln, die alle Anforderungen an eine effiziente Rheuma- oder Schmerztherapie erfüllt.

Es ist zwar bereits vorgeschlagen worden, Ibuprofen in vielfältig modulierbarer Art und Weise über einen bestimmten Zeitraum aus geeigneten Arzneiformen verzögert freizusetzen (EP 0 234 670, US-Pat. 5 004 895, EP 0 267 321), z.T. sogar mit einer Kinetik annähernd nullter Ordnung, es ist aber nicht anzunehmen, daß üblicherweise in-vitro gemessene Freigabe-Zeit-Profile mit der in-vivo Resorption des Ibuprofen in Korrelation gebracht werden können. Daher gelingt es nur ungenügend, Ibuprofen unmittelbar in resorptionsfähiger Form an den verschiedenen Resorptionsorten des Gastrointestinaltrakts zur Verfügung zu stellen. Die Folge ist nun einerseits, daß eine konstante Anflutung des Wirkstoffs in der Biophase nach Applikation einer Retardformulierung nicht sichergestellt werden kann und andererseits ein Wirkeintritt zeitlich nicht oder nur sehr schwierig vorherbestimmbar wird.

Obiges wird besonders deutlich, wenn man die gastrointestinale Passage einer konstant (nullter Ordnung) freisetzenden, Ibuprofen enthaltenden Retardarzneiform verfolgt. Analoges gilt für die Ibuprofenenantiomere S- und R-Ibuprofen, sowie für Gemische mit unterschiedlichen Anteilen S- und R-Ibuprofen. Nach heute allgemein akzeptierter Theorie verläuft die Wirkstoff-Resorption überwiegend nach den Gesetzen der passiven Diffusion, d.h. nur gelöste und gleichzeitig undissoziierte Wirkstoffmoleküle werden resorbiert. Die schwache, schwer wasserlösliche Wirkstoffsäure Ibuprofen wird im Magen-Milieu (pH 1) weitestgehend undissoziiert und kristallin vorliegen. Eine nennenswerte Resorption ist daher nicht zu erwarten. Bekannte galenische Maßnahmen zur Löslichkeitserhöhung (Solubilisation, Komplexbildung) sind nicht zu empfehlen. Gleiches gilt für das Mikronisieren von Wirkstoffen, wobei eine höhere Lösungsgeschwindigkeit durch Vergrößerung der effektiven Stoffoberfläche erzwungen werden soll. Löslich gemachte Anteile können rekristallisieren, was einerseits zu Schleimhautirritationen führen kann, andererseits ist dieser Wirkstoffanteil dann endgültig für eine Magenresorption verloren.

Schwankende Magenverweilzeiten von peroralen Retardarzneiformen verhindern zusätzlich, daß eine Wirkstoffdosis einen günstigeren Resorptionsort erreicht. So ist eine Schwankungsbreite von 0,5-10 h keine Seltenheit. Nahrungsaufnahme sowie Art und Menge der Nahrung, die Größe und Dichte der Arzneiform etc. haben entscheidenden Einfluß. Die Freisetzung des Wirkstoffs läuft aber in dieser Zeit kontinuierlich weiter. Das verdeutlicht insbesondere, daß eine ausreichende Anflutung in der Biophase nicht erwartet werden kann, es resultieren subtherapeutische Plasmaspiegel. Gleichzeitig steigt das Risiko u.U. toxischer gastrointestinaler Nebenwirkungen erheblich an.

Erst nach Erreichen des oberen Dünndarms (sprunghafter pH-Anstieg) ist genügend Ibuprofen löslich, sodaß die Resorption der undissoziierten Form in ausreichendem Maße beginnen kann. Je weiter man nun die Darmpassage verfolgt, desto höher wird der pH-Wert ansteigen (bis ca. 8). Entsprechend wird auch mehr Ibuprofen in dissoziierter Form vorliegen und der Resorptionsprozeß mehr oder weniger stagnieren. Im Dickdarm kann darüberhinaus neben der Wirkstoffauflösung die Diffusion innerhalb des weiten Darmlumens bis zur Darmwand zu einem weiteren geschwindigkeitsbestimmenden Schritt werden. Deshalb sollte gerade in diesem Darmabschnitt die Freisetzung und Auflösung des Wirkstoffs trotz der im Vergleich zum Dünndarm geringen Flüssigkeitsströme gewährleistet sein. Dies ist bei herkömmlichen Retardformulierungen meistens nicht der Fall.

Damit wird deutlich, daß man für Ibuprofen ein sogenanntes in-vivo Resorptionsfenster zu beachten hat. Darüberhinaus kann die Resorption auch starken interindividuellen Schwankungen unterworfen sein.

Eine diskutierte Alternative stellt das Prodrug-Konzept dar. Durch gezielte chemische Veränderung des Wirkstoffmoleküls hofft man, neue Ansätze für eine mindestens teilweise Umgehung der Resorptionsproblematik zu finden. Die tatsächliche Realisierung ist jedoch nur sehr schwierig zu bewerkstelligen. Prodrugs oder bioreversible Molekülvariationen sind neue Wirkstoffe (new-entities) deren pharmakologische und pharmakokinetische Eigenschaften im Vergleich zum ursprünglichen Wirkstoff völlig verändert sein können. Neue, aufwendige Untersuchungen hierzu werden notwendig.

Aus dem bisher Geschilderten wird klar, daß heute darüber diskutiert wird, ob der perorale Applikationsweg von Retardformen für alle Wirkstoffe überhaupt sinnvoll ist. Diese Tatsache wird besonders bei Ibuprofen-Racemat, reinem S- oder R-Ibuprofen und den Mischungen aus unterschiedlichen Enantiomeranteilen deutlich. Eine Hauptvoraussetzung für die Erzielung konstanter Plasmaspiegel, nämlich die kontinuierliche Resorption von freigesetztem Wirkstoff aus der Arzneiform, ist so nicht gegeben bzw. kann mit konventioneller Galenik nicht gewährleistet werden.

J.J. Marty et al., Pharm. Acta Helv. 53, 1 (1978) S. 17-23 beschreibt die Herstellung von Gelatine-Nanopartikeln, in die auch Wirkstoffe eingeschlossen werden können. Bei der Herstellung dieser Gelatine-Nanopartikel wird zur Desolvatation und Resolvatation eine pH-Justierung vorgesehen. Eine Überführung des Arzneimittels in Nanopartikel wird nicht offenbart.

Der vorliegenden Erfindung liegt daher die Aufgabe zugrunde, eine Arzneiform für Ibuprofen bereitzustellen, die für retardierte Arzneistofffreigabe geeignet ist, und die die oben zum Stand der Technik genannten Nachteile weitgehend vermeidet.

Diese Aufgabe wird erfindungsgemäß durch ein Retard-Arzneimittel gemäß Patentanspruch 1 gelöst. Diese Aufgabe wird weiterhin durch die Verwendung eines pharmazeutisch applizierbaren Nanosols von Ibuprofen gemäß Patentanspruch 17 bis 19 gelöst.

Diese Aufgabe wird ferner durch ein Arzneimittel gemäß Anspruch 11 gelöst, bei dem das Ibuprofen teilweise als Retardform, teilweise als Akutform vorliegt.

Übliche Einzeldosierungen für das Ibuprofen-Racemat liegen bei 200 mg bis 800 mg, im Falle des S-Ibuprofens sind 50 mg bis 400 mg üblich. Ibuprofen liegt im Rahmen der vorliegenden Erfindung entweder als Racemat, als racemisches Gemisch mit seinen Enantiomeren, als Pseudoracemat (Mischung von gleichen Anteilen S- und R-Ibuprofen) oder in Mischung unterschiedlicher Anteile von S- und R-Ibuprofen im Bereich zwischen reinem S- und reinem R-Ibuprofen vor.

In der internationalen (PCT-)Patentanmeldung vom heutigen Tage mit dem Titel "Pharmazeutisch applizierbares Nanosol und Verfahren zu seiner Herstellung" der ALFATEC-Pharma GmbH entsprechend der deutschen Patentanmeldung P 41 40 195.6 vom 5.12.1991, deren Inhalt auch zum Inhalt der vorliegenden Patentanmeldung gemacht wird, werden Nanosole und Verfahren zu ihrer Herstellung beschrieben, die es ermöglichen, kolloiddisperse Lösungen von in Wasser schwer löslichen Wirkstoffen durch Gelatine, Kollagenhydrolysate oder Gelatinederivate zu stabilisieren, wenn man den isoionischen Punkt (=Ladungsausgleich) zwischen Gelatine und den auf der Oberfläche geladenen Wirkstoffpartikeln wenigstens annähernd einstellt. Dabei bringt man das System Wirkstoffpartikel/Gelatine dadurch zum Ladungsausgleich, daß die Oberflächenladung der Partikel durch entsprechende Gegenladung der Gelatinemoleküle kompensiert wird. Erreicht wird dies durch Einstellung einer bestimmten Ladung auf den Gelatinemolekülen, die in Abhängigkeit zu ihrem isoelektrischen Punkt und dem pH-Wert der Lösung steht.

Fig. 1 zeigt die Auflösungsprofile von einer erfindungsgemäßen Tablette (Bsp. 1) und von einer herkömmlichen Filmtablette,

Fig. 2 zeigt die Plasmakonzentrations-Zeitverläufe von einer erfindungsgemäßen Nanosol-Tablette und von einer wäßrigen Suspension

Fig. 3 zeigt eine schematische Darstellung der einstellbaren Ladungszustände von Gelatine in Abhängigkeit vom pH-Wert und IEP, wobei der IEP je nach Herstellungsart zwischen 3,5 und 9,5 liegen kann. Unterhalb von pH 3,5 sind fast alle Gelatinetypen positiv geladen. Im basischen Bereich oberhalb von pH 9,5 sind alle Gelatinetypen negativ geladen.

Fig. 4 zeigt den Mechanismus der passiven Arzneistoff-Resorption im Gastrointestinal-Trakt.

Erfindungsgemäß wird daher die Tatsache ausgenutzt, daß Gelatinen, Kollagenhydrolysate oder Gelatinederivate (nahezu unabhängig von der Viskosität) dann zu einem stabilen kolloid-dispersen Systems in Nanosolform führen, wenn der isoionische Ladungszustand zwischen Arzneistoffpartikel und Gelatine, Kollagenhydrolysat oder Gelatinederivat vorliegt.

Dagegen wurde Gelatine nach dem Stand der Technik nur zur Stabilisierung eines anorganischen, kolloid-dispersen Systems eingesetzt. So beschreibt z.B. das DAB 9 eine kolloidale Injektionslösung von radioaktivem Gold, die mit Gelatine hergestellt ist. Man stellte sich dabei lediglich vor, daß sich Makromolekül als "Kittsubstanz" zwischen den einzelnen Kolloidpartikeln befinde und so eine Teilchenaggregation verhindert werde.Dagegen war über den Stabilisierungsmechanismus, z.B. für Arzneistoffe, bisher nichts bekannt.

Die internationalen (PCT-) Patentanmeldungen vom heutigen Tage der ALFATEC-Pharma GmbH und der PAZ Arzneimittelentwicklungsgesellschaft mbH entsprechend der genannten deutschen Patentanmeldung (vom 5.12.1991) betreffen die Akutform von S- und R-Ibuprofen
(P 41 40 179.4), die Retardform von S- und R-Ibuprofen
(P 41 40 172.7), die Akutform von S- und R-Flurbiprofen
(P 41 40 184.0) und die Retardform von S- und R-Flurbiprofen
(P 41 40 183.2). Ihre Offenbarung wird auch zum Gegenstand der vorliegenden Patentanmeldung gemacht.

So bietet sich für die Rheumatherapie eine völlig neue Kombination aus Akut- und Retardform auf Nanosolbasis an: Akut-Ibuprofen und Retard-Ibuprofen.

Um die physiologischen Hintergründe der Resorption von Arzneistoffen im allgemeinen und die verbesserte Resorptionsquote der erfindungsgemäßen Nanosole ausreichend zu erläutern, ist zunächst eine Betrachtung zum Mechanismus der physiologischen Resorption von Arzneistoffen erforderlich, wie er auch in einschlägigen Publikationen dargestellt wird. Allerdings ist die vorliegende Erfindung weder an den folgenden Versuch einer wissenschaftlichen Erklärung der erfindungsgemäß auftretenden Phenomene gebunden noch kann sie hierdurch eingeschränkt werden.

Die passive Arzneistoffresorption erfolgt nach heutigem Erkenntnisstand (Theorie nach Brodie et al.), wenn folgende Bedingungen vorliegen:

a) die Gastrointestinalmembran wirkt als Lipidbarriere,
b) der Arzneistoff wird nur in gelöster und ungeladener, d.h. nichtionisierter Form aufgenommen,
c) saure Arzneistoffe werden bevorzugt im Magen, basische Arzneistoffe bevorzugt im Darm resorbiert.

Nach der peroralen Aufnahme eines Arzneistoffs in den Organismus wird seine Resorption, d.h. der Übertritt in den allgemeinen Kreislauf (Biophase) in starkem Maße durch physikalische Barrieren behindert (siehe Abb. 2), nämlich

- durch die Mucus-Schicht und eine wässerige, daran adhärierende Schicht
- die Zellmembranen der intestinalen Epithelzellen mit der daran kovalent gebundenen Glykocalix sowie
- die sogenannten "Tight Junctions", die die Epithelzellen an ihrer apikalen Seite miteinander verbinden.

Diese Barrieren bedingen, daß die Resorption von Arzneistoffen hauptsächlich abhängig von ihrem Verteilungsmechanismus und Ladungszustand- durch die Lipid-Doppelschichten erfolgt (sogenannte passive Diffusion).

Die Epithelzellen des gesamten Magen-Darm-Traktes sind mit einer Mucus-Schicht bedeckt, die aus Mucinen (Glykoproteinen), Elektrolyten, Proteinen und Nucleinsäuren besteht. Vor allem die Glykoproteine bilden mit dem Hauptanteil des Mucus, nämlich Wasser, eine viskose Gelstruktur, die in erster Linie Schutzfunktionen für die darunter liegende Epithelschicht ausübt. Die Mucusschicht ist an die apikale Oberfläche der Epithelzellen über die Glykocalix gebunden. Die Glykocalix hat ebenfalls eine Glykoproteinstruktur, die kovalent an Bausteine der Membran-Doppelschicht der Epithelzellen gebunden ist. Die verzweigten Polysaccharide der Glykocalix, die entweder direkt an amphiphile Moleküle der Doppelmembran oder an die Doppelmembran inkorporierte Proteine kovalent gebunden sind, besitzen geladene N-Acetyl-Neuraminssäure- und Sulfat-Reste und sind daher negativ geladen, was zu einer elektrostatischen Bindung oder Abstoßung von geladenen Arzneistoffmolekülen bzw. von elektrostatisch geladenen Partikeln führen kann. Die Epithelzellmembranen bestehen aus Phospholipid-Doppelschichten, in die Proteine über ihre hydrophoben Bereiche verankert sind. Die Phospholipid-Doppelschichten mit ihrem lipophilen Anteil stellen eine weitere Barriere für den Transport der zu resorbierenden Arzneistoffe dar.

Aus dieser Darstellung geht deutlich hervor, daß geladene Arzneistoffmoleküle bzw. elektrostatisch geladene Partikel daher nur eine sehr geringe Chance haben, über den peroralen Applikationsweg resorbiert zu werden.

Die erfindungsgemäßen Nanosole geben erstmalig die technische Lehre, ein System zu bilden, mit dem diese vorgenannten Resorptionshindernisse zu überwinden sind. Da die Wirkstoff-Nanopartikel durch die Gelatine erfindungsgemäß ladungsneutral stabilisiert werden, kann ihr Transport durch die negativ geladene Glykocalix ohne größere Hindernisse erfolgen, im Gegensatz zu sonstig beschriebenen Nanopartikeln des Standes der Technik, die nicht ladungsneutral stabilisiert werden bzw. stabilisiert werden können. Erfindungsgemäß kann die Einstellung des isoionischen Ladungszustandes zusätzlich noch in Abstimmung auf die physiologischen Verhältnisse erfolgen.

Da die erfindungsgemäßen Wirkstoff-Nanosole die Glykocalix ungehindert passieren können, ohne durch elektrostatische Effekte gebunden bzw. abgestoßen zu werden, erreichen sie damit auch die

Oberfläche der Epithelzellen und stehen dort in hoher Konzentration zur Verfügung.

Nun können auch aktive, carriervermittelte Transportmechanismen bzw. Phagozytose einen wesentlichen Beitrag zur Resorption der Wirkstoff-Nanosole liefern.

Im Falle von S-Ibuprofen, insbesondere bei höherer Dosierung sind bevorzugt Gelatinesorten geeignet, die einen Anteil an rechtsdrehenden Aminosäuren unterhalb von 20% aufweisen und deren Maximum der Molekulargewichtsverteilung unterhalb $10^5$ D liegt. Zur Tablettenherstellung, wie sie üblicherweise bei Schmerzmitteln im Vordergrund steht, eignen sich bevorzugt Gelatinesorten mit Bloomwerten von 0 - 50. Bei den genannten Gelatinen kann vorteilhafterweise ein Gewichtsverhältnis Gelatine zu Wirkstoff von 0,5:1 bis 3:1 eingehalten werden.

Bei der Formulierung von Akut- bzw. Retardpräparaten macht der Pharmazeut einen grundsätzlichen Unterschied zwischen:

1. galenischer Zubereitung, d.h. einer Freisetzung des Arzneistoffes, z.B. aus einer Tablette in zeitlich schneller (Akutform) oder verlangsamter (Retardform) Weise;
und
2. dem arzneistoffspezifischen Resorptionsort, wie z.B. Magen oder bestimmte Darmabschnitte.

Die erfindungsgemäßen Nanosole sind in der Lage, unabhängig von der galenischen Zubereitung, aufgrund ihrer speziellen Zusammensetzung, im gesamten gastrointestinalen Bereich resorbiert zu werden. Sie können daher vorteilhaft zu Akut- bzw. Retardarzneiformen weiterverarbeitet werden.

Erfindungsgemäß kann ein Ibuprofen als Racemat oder als racemisches Gemisch mit seinen Enantiomeren eingesetzt werden. Es eignet sich auch ein aus 50 % S-Ibuprofen und 50 % R-Ibuprofen synthetisch zusammengesetztes Pseudoracemat. Weiterhin lassen sich die reinen S- oder R-Enantiomeren einsetzen. Schließlich kann das Ibuprofen auch als Gemisch mit unterschiedlichen Anteilen an S- und R-Enantiomeren vorliegen.

Überraschenderweise zeigt sich, daß das Vorliegen stabiler Nanopartikel im Falle des schwer wasserlöslichen Ibuprofen-Racemats oder seiner Enantiomere völlig ausreichend ist, eine Arzneistoffresorption im gesamten Gastrointestinaltrakt (GIT) -auch im Magen- zu erreichen, die

a) unabhängig von den oben geschilderten physiologischen Bedingungen ist
b) unabhängig von den physikalisch-chemischen Eigenschaften des Ibuprofens ist
c) nahezu vollständig ist und
d) ohne vorgelagertes Gleichgewicht der Wirkstoffauflösung erfolgt wie bei herkömmlichen Retardformen (der Wirkstoff steht in resoptionsfähiger Form unmittelbar an jedem beliebigen Resorptionsort zur Verfügung).

Offensichtlich werden Nanosole nach einem bisher nicht bekannten Mechanismus resorbiert, wie es z.B. in der parallelen internationalen (PCT)-Patentanmeldung "Akutform für ein Ibuprofen enthaltendes Arzneimittel und seine Herstellung", vom heutigen Tage, entsprechend der deutschen Patentanmeldung P 40 179.4 für die überraschende Resorption im Magen in vivo gezeigt wird.

Betrachtet man nun die gastrointestinale Passage einer geeigneten Retardformulierung, die ein Ibuprofen-Nanosol enthält, das während dieser konstant freigegeben wird, so ergibt sich ein völlig neuartiges Bild:

Unmittelbar auf die Freigabe der Nanopartikel aus der Arzneiform im Magen erfolgt deren Resorption. Es ist dabei gleichgültig, wie lange die Formulierung selbst im Magen verweilt. Schwankende Magenverweilzeiten von z.B. single-unit Retardarzneiformen müssen nicht mehr berücksichtigt werden, wie bei konventioneller Galenik. Damit wird ein Wirkungseintritt nach Applikation zeitlich vorhersagbar.

Eine besonders geeignete galenische Retardformulierung wird in der gleichzeitig angemeldeten internationalen (PCT)- Patentanmeldung "Sol-gesteuerte Thermokolloidmatrix auf Gelatinebasis für perorale Retardarzneiformen" der ALFATEC-Pharma GmbH entsprechend der deutschen Patentanmeldung P 41 40 192.1 von demselben Tage vorgestellt, deren Inhalt auch zum Gegenstand der vorliegenden Erfindung gemacht wird.

Diese nahezu nullter Ordnung freisetzende Retardtablette mit einem Matrixmaterial aus Gelatine, hat sich erstaunlicherweise dadurch ausgezeichnet, daß sie die Verteilung der eingebetteten, erfindungsgemäßen Nanopartikel auf der Schleimhautoberfläche gleichmäßig gewährleistet. Somit sind die erfindungsgemäßen Nanopartikel auch wirksam vor den Einflüssen von Nahrung und Nahrungsbestandteilen geschützt.

Auch nach der Magenpassage, verbunden mit einem pH-Anstieg im oberen Dünndarm bis hin zum Dickdarm ist keine Einschränkung der Resorptionsfähigkeit der Nanopartikel in den erfindungsgemäßen Nanosolen zu befürchten. Man sollte eigentlich annehmen, daß die Stabilität der Nanosole bei pH-Werten, die oberhalb des $pK_A$-Werts von Ibuprofen liegen, rapide absinkt bzw. die Ibuprofen-Nanopartikel sich rasch durch Dissoziation auflösen. Dem ist jedoch nicht so.

Einerseits können die erfindungsgemäßen Nanosole durch Selektion von Gelatinesorten, die mit den Ibuprofen-Nanopartikeln im weiter sauren Bereich, z.B. im pH-Bereich von 1,5 - 2,5 den isoionischen Punkt

erreichen, hergestellt werden. Die Nanopartikel sind damit wirksam vor der Einwirkung eines schwächer sauren bis alkalischen Milieus geschützt. Die oben angesprochene, zeitliche Auflösung der Nanopartikel bei physiologischen pH-Werten, die oberhalb des $pK_A$-Werts von Ibuprofen liegen, ist dadurch mindestens bis zur Resorption vermindert oder sogar gänzlich verhindert.

Andererseits kann durch die erfindungsgemäße Einbettung der Ibuprofen-Nanosole in die Thermokolloidmatrix, die sich während der Gastrointestinalpassage kontrolliert auflöst, eine über mehrere Stunden nahezu konstante Resorptionsquote erzielt werden.

Denkbar als solche Matrixarzneiformen sind Matrixtabletten allein oder z.B. abgefüllt in Hartgelatinekapseln mit Akut-Ibuprofen auf Nanosolbasis. In der Matrix selbst können auch puffernde Hilfsstoffe vorliegen, die die erfindungsgemäßen Nanopartikel bzw. das Nanosol wirksam vor physiologischen pH-Schwankungen schützen. Das die Nanopartikel bzw. Nanosol umgebende Milieu kann dadurch pH-stabil fixiert werden.

Weiterhin hat sich gezeigt, daß auch im Darm die Gelatine in den erfindungsgemäßen Nanosolen die gleichmäßige Verteilung der Nanopartikel auf der Darmschleimhaut ermöglicht. Da an der Schleimhautoberfläche, im Vergleich zum Darmlumen ein schwach saurer pH-Wert vorherrscht, sind dort die erfindungsgemäßen Nanopartikel bis zur Resorption noch effektiver vor pH-Schwankungen geschützt.

Durch alle die genannten Vorzüge gemeinsam lassen sich bei Ibuprofen Bioverfügbarkeiten erreichen, wie sie bisher nicht bekannt sind. Damit verbunden ist ebenso eine Verkürzung der Zeit von der Applikation bis zum Erreichen der Plasmawirkstoffkonzentration im therapeutischen Niveau (steady-state), als auch eine geringe Schwankungsbreite des Plasmaspiegels. Außerdem wird die in der erfindungsgemäßen Arzneiform enthaltene Wirkstoffdosis vollständig ausgenutzt, sodaß damit insgesamt gesehen die analgetisch/antiphlogistische Wirkung gegenüber konventionellen Retardformen deutlich verbessert und die Verträglichkeit erhöht wird.

Erstaunlicherweise hat sich gezeigt, daß diese Nanopartikel in dem erfindungsgemäßen Nanosol an jedem gewünschten Resorptionsort ungehindert die Gastrointestinalmembran passieren können (resorbiert werden). Sie verhalten sich also, biopharmazeutisch gesehen, wie eine echte Lösung, ohne aber eine solche zu sein.

Für peroral anzuwendende Retardformen ist bisher nichts derartiges bekannt.

Da in den erfindungsgemäßen Nanosolen Teilchenwachstum durch Ostwald-Reifung wirksam vermindert wird (siehe Ausführungen der o.g. Anmeldung P 41 40 195.6 der ALFATEC-Pharma GmbH), ist keine Einschränkung der Resorptionsfähigkeit der Nanopartikel zu befürchten.

Es hat sich weiterhin gezeigt, daß nur Nanosole, deren Größe unter 800 nm liegt, bevozugt im Bereich von 10 nm bis 600 nm, insbesondere aber im Bereich unterhalb von 400 nm vollständig und schnell resorbiert werden können.

Prinzipiell sind zur Herstellung der erfindungsgemäßen Nanosole die in der o.g. deutschen Patentanmeldung P 41 40 195.6 der ALFATEC-Pharma GmbH "Pharmazeutisch applizierbares Nanosol und Verfahren zu seiner Herstellung" genannten Vorgehensweisen und Verfahrensvarianten geeignet, die im folgenden noch einmal angeführt werden:

Es werden mehrere Verfahren zur Herstellung der Nanosole vorgeschlagen. Dabei handelt es sich um eine beispielhafte, unvollständige Aufzählung. Der Fachmann kann aufgrund seines Fachwissens selbstständig weitere Varianten im Rahmen der vorliegenden Erfindung ausarbeiten:

## Verfahren I

Dieses kann angewendet werden, wenn der Arzneistoff in einer Mischung aus:
einem mit Wasser mischbaren organischen Lösungsmittel und Wasser, oder
aus mehreren mit Wasser mischbaren organischen Lösungsmitteln und Wasser löslich ist:

a) eine in den Vorversuchen ausgewählte Gelatine wird mit Wasser in Solform überführt;
b) der in den Vorversuchen gefundene pH-Wert der Lösung wird eingestellt;
c) ein oder mehrere mit Wasser mischbare(s), organische(s) Lösungsmittel, vorzugsweise Ethanol, Isopropanol oder Methanol, wird/werden zu dieser Lösung gegeben;
d) der Arzneistoff wird in fester Form zu der Lösung gegeben und gelöst;
e) das/die organische(n) Lösungsmittel wird/werden entfernt, vorzugsweise durch Eindampfen in Vakuum; dabei entsteht das Nanosol;
f) die kolloid-disperse Lösung wird anschließend, vorzugsweise durch Sprüh- oder Gefriertrocknung, getrocknet.

Das organische Lösungsmittel hat die Aufgabe, den Arzneistoff zu lösen und verändert auch die Hydrathülle der Gelatinemoleküle.

## Verfahren II

Diese Ausführungsform kann angewendet werden, wenn der Arzneistoff eine Säure oder eine Base ist, deren Salz in Wasser löslich ist:

a) eine in den Vorversuchen ausgewählte Gelatine wird mit $H_2O$ in die Solform überführt;

b) es wird ein solcher pH-Wert eingestellt, der die Salzbildung des Arzneistoffs ermöglicht;

c) der Arzneistoff wird unter Salzbildung in dem Gelatinesol gelöst;

d) durch Zugabe von Alkohol oder ähnlichen organischen Lösungsmitteln kann die Hydrathülle der Gelatinemoleküle gelockert werden;

e) durch Zugabe einer geeigneten Menge Säure oder Base wird der pH-Wert eingestellt, der zur Bildung des isoionischen Punkts (IIP) führt, dabei entsteht das Nanosol;

f) die kolloid-disperse Lösung wird wie in Verfahren I getrocknet.

Stufe d) ist fakultativ, jedoch bevorzugt.

## Verfahren III

Diese Ausführungsform kann angewendet werden, wenn der Arzneistoff ein Neutralstoff ist:

a) es wird ein Gelatinesol hergestellt, wie unter (1) a) und b) beschrieben.

b) eine zweite Lösung aus einem mit Wasser mischbaren organischen Lösungsmittel, vorzugsweise Ethanol, Methanol, Isopropanol, Aceton und dem Arzneistoff wird hergestellt.

c) die beiden Lösungen werden vereinigt.

d) das organische Lösungsmittel wird entfernt und die kolloid-disperse Lösung wird getrocknet.

## Verfahren IV

a) Wie unter (I) a) und b) beschrieben.

b) In einer zweiten Lösung wird ein kolloid-disperses System mit dem Arzneistoff kurzzeitig gebildet, jedoch ohne Gelatine.

c) Die unter (b) erhaltene Lösung wird kontinuierlich mit der Gelatinelösung vereinigt.

Bei Schritt (IV) c) kann die kontinuierliche Vermischung der unter (IV) a) und b) beschriebenen Lösungen zeitabhängig durch on-line Messung der Teilchengröße mit einem geeigneten Verfahren, wie z.B. durch Laser-Licht-Streuung (BI-FOQELS On-line Particle Sizer), gesteuert werden. Damit ist es möglich, eine gewünschte Partikelgröße kontinuierlich einzustellen.

Alle genannten Verfahren sind auch für Kollagenhydrolysate und Gelatinederivate geeignet und können problemlos in den technischen Maßstab übertragen werden.

Die wesentlichen Schritte können weitgehend automatisiert ablaufen, wobei auch Verfahren I bis III kontinuierlich durchführbar sind. Im Falle der Akutform für 2-Arylpropionsäurederivate seien als bevorzugt geeignete Verfahren die Varianten Nr. II und III genannt.

Für die erfindungsgemäßen Akutformen eignen sich alle Gelatinen, Gelatinederivate, Kollagenhydrolysate und fraktionierte Gelatine, sowie deren Mischungen. Gelatinesorten, die einen erfindungsgemäß beschriebenen isoelektrischen Punkt (IEP) aufweisen, der nicht handelsüblich ist, können nach den Beispielen I bis III aus o.g. deutscher Patentanmeldung hergestellt werden.

Gegenüber handelsüblichen Produkten führt die Verwendung von Gelatine, die auf spezielle Weise hergestellt wurde, zu erfindungsgemäß beschriebenen Nanosolen mit erhöhter Stabilität.

Beispiele für die Herstellung erfindungsgemäß besonders geeigneter Gelatinequalitäten werden unten gegeben.

**Beispiele für die Herstellung von erfindungsgemäß besonders geeigneten Gelatinesorten mit isoelektrischen Punkten von 3,5 bis 9,5**

Beispiel I:

**Verfahren zur Erzielung eines IEP's von 7,5 bis 9,5**

Kollagenhaltiges Ausgangsmaterial wie z.B. Schweineschwarten werden mit einer wäßrigen Lösung einer 0,45 N Mineralsäure, vorzugsweise Schwefelsäure, im Flottenverhältnis 1:1 12 bis 20 Stunden behandelt. Anschließend wird der Säureüberschuß durch mehrmaliges Waschen entfernt, wobei zur Abkürzung des Verfahrens Natriumhydrogencarbonat verwendet werden kann. Die Extraktion des sudreifen

Materials erfolgt mit heißem Wasser bei 55 - 80° C bei einem pH von 2,5 bis 4,5. Bei pH-Werten unterhalb von 3,5 kann ein IEP von 8,5 bis 9,5 erreicht werden, bei pH-Werten oberhalb 3,5 liegt der IEP bei 7 bis 8,5. Auf diese Weise lassen sich verschiedene IEP's von 7 bis 9,5 in direkter Abhängigkeit vom pH-Wert während der Extraktion erzielen.

Nach der Verfahrensstufe der Extraktion wird die wäßrige Lösung neutralisiert und wie üblich aufgearbeitet.

Durch dieses Verfahren kann man weiterhin in Abhängigkeit von der gewählten Temperatur während der Extraktion Gelatinesorten mit hohen bis mittleren Molekulargewichtsverteilungen erhalten.

Bei Temperaturen von 50-55° C erhält man besonders hochviskose und hochbloomige Qualitäten. Gelatinesorten mit niedrigem Molekulargewicht bzw. kaltwasserlösliche Gelatinen können durch gezielten Abbau mit Kollagenasen erhalten werden.

Beispiel II:

**Verfahren zur Erzielung eines IEP's von 4 bis 7,5**

Das kollagenhaltige Ausgangsmaterial wird zur Entfernung von Fremdstoffen zunächst gewaschen, zerkleinert und anschließend durch Zusatz von Magnesit, Natronlauge oder Calciumhydroxid durch gründliches Vermischen im Flottenverhältnis 1:1,2 homogen alkalisch gemacht. Das so vorbehandelte Material wird kurzzeitig druckhydrolytisch bei $1{,}01 \times 10^5$ bis $2{,}02 \times 10^5$ Pa und einem pH-Wert der wäßrigen Lösung von 8-14 aufgeschlossen. Nach dem Aufschluß wird sofort neutralisiert und die noch heiße wäßrige Gelatinelösung wie üblich filtriert, entsalzt, aufkonzentriert und getrocknet.

Nimmt man ein schwach basisches Aufschlußmittel wie Magnesit, erhält man einen IEP von 6 bis 7,5, sofern man bei $1{,}01 \times 10^5$ Pa arbeitet. IEP's von 5 bis 6 erhält man bei Einsatz einer verdünnten Kalkmilchsuspension und bei Verwendung von 0,005 bis 0,1 N Natronlauge können IEP's von 4 bis 5 erzielt werden.

Gelatinesorten mit geringem Racemisierungsgrad und niedrigem Peptidanteil lassen sich bei Druckverhältnissen von $1{,}01 \times 10^5$ Pa und Verweilzeiten von maximal 10 Min. erreichen.

Mittel- bis niedrigmolekulare bis hin zu kaltwasserlöslichen Sorten ergeben sich durch entsprechend längere Verweilzeiten.

Beispiel III:

**Verfahren zur Erzielung eines IEP's von 3,5 bis 6**

Kollagenhaltiges Ausgangsmaterial, vorzugsweise Spalt bzw. Ossein, wird nach der Eingangswäsche einem Kurzzeitäscher unterworfen. Hierbei bieten sich zwei Verfahrensvarianten im Flottenverhältnis 1:1,3 an, die entweder eine gesättigte Kalkmilchsuspension oder eine 0,1 bis 1 N Natronlauge zum Einsatz bringen.

Bei Verwendung einer Kalkmilchsuspension wird das Rohmaterial unter ständiger Bewegung maximal 3 bis 4 Wochen aufgeschlossen. Anschließend wird das Material durch Säurezugabe neutralisiert und mehrmals gewaschen. Die weitere Aufarbeitung folgt wie üblich. Auf diese Weise lassen sich IEP's von 4 bis 6 einstellen.

Bei Einsatz von Natronlauge läßt sich der Äscherprozeß nochmals verkürzen, wobei bei Konzentrationen von 1 N Natronlauge das Material je nach Zerkleinerungsgrad bereits nach 6 - 12 Stunden aufgeschlossen ist. Die Neutralisation erfolgt mit äquimolaren Mengen Mineralsäure und die Neutralsalze werden durch mehrmaliges Waschen oder durch Entsalzen der in der Extraktion gewonnenen wäßrigen Gelatinelösung entfernt. Bei dieser Verfahrensvariante lassen sich IEP's von 3,5 bis 5 erhalten.

Besonders peptidarme Gelatinesorten werden bei kurzer Verweilzeit im Äscher erhalten. Man kann so Gelatinesorten mit hoher bis mittlerer Molekulargewichtsverteilung ($M = 10^4 - 10^7$ D) erhalten.

Niedrigmolekulare bis kaltwasserlösliche Gelatinesorten kann man durch thermischen Abbau bzw. enzymatisch erhalten.

Bevorzugt werden im Falle der 2-Arylpropionsäurederivate Gelatinesorten mit IEP von 3,5 bis 9,5 eingesetzt.

Übliche pharmazeutische Hilfsstoffe und/oder weitere Makromoleküle können, sofern sie technologisch erforderlich sind, in flüssigem oder getrocknetem Zustand den erfindungsgemäßen Nanosolen zugesetzt werden.

Zum Beispiel kann ein Zusatz von Polyvinylpyrrolidon im Mengenverhältnis Gelatine zu Polyvinylpyrrolidon im Bereich von 5:1 bis 500:1 geeignet sein.

Eine Akutform im Sinne der Erfindung, die z.B. zu Tabletten verarbeitet wird oder lyophilisiert werden soll, kann durch Zusatz von niedrigmolekularen Polyvinylpyrrolidonsorten im Bereich von 10:1 bis 50:1 in den technologischen Verarbeitungseigenschaften verbessert werden, ohne daß die Stabilität der Nanosole negativ beeinflußt wird.

Die in den folgenden Beispielen bevorzugten Herstellungsverfahren, Vorgehensweisen und Bezeichnungen beziehen sich wie folgt auf die deutschen Patentanmeldungen "Pharmazeutisch applizierbares Nanosol und Verfahren zu seiner Herstellung" (P 41 40 195.6) bzw. die oben genannten Verfahren und Beispiele:

| | |
|---|---|
| Nanosol-Herstellung: | Verfahren II und III |
| Gelatineherstellung: | Beispiel I bis III |
| Vortest: | siehe folgende Beschreibung: |

Vortest:

Wie eingangs schon erwähnt und wie aus Fig.1 ersichtlich ist, hängt die absolute, maximal mögliche Nettoladung eines einzelnen Gelatinemoleküls hauptsächlich von der Anzahl der freien COOH- und $NH_2$-Gruppen und dem pH-Wert der Lösung ab.

Da sich Typ A, B, Kollagenhydrolysate oder Gelatinederivate in der Anzahl freier COOH-Gruppen unterscheiden, ist damit auch ihre maximal mögliche Nettoladung unterschiedlich. Bei Gelatinederivaten kann der Ladungszustand zusätzlich von der Art der Modifizierung abhängen.

Bei der Durchführung des erfindungsgemäßen Verfahrens wählt man in einem Vortest die geeignete Gelatine und den geeigneten pH-Wert aus.

Zunächst wird ein den physikalisch-chemischen Eigenschaften des Arzneistoffs angepaßter Arbeits-pH-Bereich gewählt. Als physikalisch-chemische Eigenschaft des Arzneistoffs sind vor allem zu berücksichtigen: Die Löslichkeit (in organischen Lösungsmitteln bzw. Wasser), seine Eigenschaft als Säure, Base oder Neutralstoff sowie seine Stabilität gegenüber Säuren und Laugen.

In einem ersten Schnelltest wird festgestellt, welche Ladung die ausgefällten Partikel besitzen. Daraus ergibt sich, unter Berücksichtigung des Arbeits-pH-Bereichs, die Wahl eines geeigneten Gelatinetyps. Sind die Teilchen beispielsweise negativ geladen, sucht man eine Gelatine aus, die unter den gegebenen pH-Bedingungen positiv geladen ist. Dieser Schnelltest zur Feststellung der Partikelladung hat die Vorteile, daß er ohne großen apparativen und zeitlichen Aufwand durchgeführt werden kann. So kann auf eine zeitaufwendige und ungenaue Zeta-Potential-Messung gänzlich verzichtet werden.

In vielen Fällen wird es ausreichend sein, für diesen Schnelltest zwei handelsübliche Gelatinen Typ A und B mit einem IEP von 9,5 bzw. 3,5 mit Peptidanteilen <30 % und einer Bloomzahl von 200, die weiterhin als Standardgelatinen bezeichnet werden, bei einem pH-Wert von 6 in die Solform zu überführen (5%ige wäßrige Lösung) und den Arzneistoff in einem mit Wasser mischbaren Lösungsmittel, wie z. B. Ethanol, Isopropanol oder Aceton, zu lösen und jeweils mit den Gelatinelösungen homogen zu mischen. Bei gleicher Dosierung des Arzneistoffs wird sich bei der in ihrem Ladungszustand nicht geeigneten Gelatine ein kolloidales System entweder nicht ausbilden oder sofort instabil werden bzw. der Arzneistoff ausflocken. Sind die entstehenden Partikel negativ geladen, werden sie eher von Gelatinelösung mit Typ A, der bei einem pH-Wert von 6 positiv geladen ist, stabilisiert als von der Lösung mit Gelatine Typ B; im Gegenteil wird in diesem Fall Typ B entweder kein kolloidales System ausbilden oder das System sofort destabilisieren. Das Ausflocken der Teilchen läßt sich z. B. über eine einfache Trübungs-Messung verfolgen.

Bei diesem Schnelltest muß auf jeden Fall der Arbeits-pH-Bereich beachtet werden. Man kann auch andere Gelatinen als Standard auswählen, sie müssen jedoch in ihrem IEP so gewählt werden, daß sie bei diesem pH-Wert entgegengesetzte Nettoladung tragen (siehe auch Fig.1). In den meisten Fällen werden die besagten Standardgelatinen Typ A und B für diesen Schnelltest ausreichen.

Ausgehend vom Ergebnis des Vorversuchs werden nun durch schrittweise Variation des IEP's durch Verwendung entsprechender Gelatinesorten und des pH-Wertes der Lösung in kleineren Bereichen (z. B. 0,1 pH-Schritte) die optimalen Bedingungen zur Bildung der Nanosole ermittelt. D.h. es muß das Stabilitätsoptimum, das durch den isoionischen Punkt (IIP) gekennzeichnet ist, gefunden werden, um eine ausreichende Stabilität für die genannten pharmazeutischen Anwendungen zu gewährleisten.

Es kann durchaus der Fall sein, daß eine im Sinne der Erfindung akzeptable Stabilität der Nanosole bereits in einem engeren pH-Bereich (ca. 0,5 Einheiten) um den isoionischen Punkt gefunden wird, so daß eine Einstellung dieses Punktes selbst nicht unbedingt notwendig ist. Andererseits können auch mehrere Gelatinen zu den gleichen, stabilen Ergebnissen führen. So kann beispielsweise (Beispiel 5) mit dem oralen Antidiabetikum Glibenclamid bei einem Gelatinetyp B mit einem IEP von 5,5 das Stabilitätsoptimum bei

einem pH-Wert von 3,2 liegen, während bei einem Gelatinetyp B mit einem IEP von 3,8 das Stabilitätsoptimum bei einem pH-Wert von 2,2 liegt.

Gekennzeichnet durch ein Stabilitätsmaximum, wurde in beiden Fällen der isoionische Punkt erreicht (die Abhängigkeit der Nettoladung vom pH-Wert und dem IEP muß nicht linear sein, da sie durch den $pK_s$-Wert der vorhandenen COOH- bzw. $NH_3^+$ -Gruppen gegeben ist).

Erfindungsgemäß können alle Gelatinesorten mit einem Maximum der Molekulargewichtsverteilung im Bereich von $10^4$ bis $10^7$ D eingesetzt werden.

Kollagenhydrolysate, fraktionierte Gelatine mit niedrigem MG, Gelatinederivate und Gelatinen mit niedrigen Bloomwerten sind dann geeignet, wenn die so hergestellten Nanosole mit geeigneten galenischen Methoden unter Zusatz von weiteren Hilfsstoffen retardiert vorliegen.

Besonders geeignet sind Gelatinesorten mit einem Peptidanteil < 5% und einem Maximum der Molekulargewichtsverteilung oberhalb von $9,5 \times 10^4$ D. Vorteilhaft können besonders hochviskose Gelatinesorten oder fraktionierte Gelatinen mit einem prozentualen Gewichtsanteil der Mikrogelfraktion (> $10^7$ D) größer als 15 Prozent eingesetzt werden.

In Abhängigkeit von der Herstellungsweise von Gelatine (Ausmaß des Abbaus des nativen Kollagens und saures bzw. alkalisches Aufschlußverfahren) weist Gelatine vom Typ A oder Typ B ein charakteristisches Molekulargewichtsspektrum bzw. Molekulargewichtsverteilung auf. In Tabelle 1 sind die Molekulargewichtsverteilungen von verschiedenen Gelatinetypen bzw. von Kollagenhydrolysaten angegeben, sowie der prozentuale Anteil (Häufigkeit) einzelner Molekulargewichtsbereiche.

Tabelle 1

| Molekulargewichtsverteilung von verschiedenen bekannten Gelatinetypen bzw. von bekannten Kollagenhydrolysaten | | | | | | | |
|---|---|---|---|---|---|---|---|
| Molecular Mass Distribution (kD) | Native Collagen % | Gelatin Type B % | Gelatin Type A % | Collagen hydrolysate Gelita ® Collagel A | Collagen hydrolysate Gelita ® Collagel B | Collagen hydrolysate Gelita ® Sol C | Elastin hydrolysate Gelita ® Gelastin |
| >360 | 100 | 18,0 | 18,0 | 0 | 0 | 0 | 0 |
| 285 | 0 | 7,0 | 9,0 | 0 | 0 | 0 | 0 |
| 145-237 | 0 | 20,0 | 34,0 | 1,0 | 1,5 | 0 | 0 |
| 95 | 0 | 26,0 | 11,0 | 0 | 0 | 0 | 0 |
| 95-50 | 0 | 16,3 | 13,4 | 2,6 | 4,0 | 1,1 | 0 |
| 50-20 | 0 | 7,4 | 9,1 | 18,0 | 14,5 | 0,3 | 0 |
| 20-10 | 0 | 3,9 | 3,8 | 43,0 | 31,5 | 3,7 | 0,2 |
| 10-5 | 0 | 3,0 | 3,0 | 15,4 | 20,0 | 12,2 | 5,2 |
| 5-2 | 0 | 0 | 0 | 6,0 | 14,0 | 26,0 | 93,9 |
| 2-1 | 0 | 0 | 0 | 7,0 | 8,0 | 23,0 | 0 |
| <1 | 0 | 0 | 0 | 6,5 | 7,0 | 34,0 | 0 |
| MG | 360 | 165 | 185 | 12-18 | 12-18 | 3 | 2-3 |

Man erkennt in den einzelnen Spalten deutlich das Überwiegen eines einzelnen Bereiches im Vergleich zu den übrigen Molekulargewichtsbereichen derselben Gelatine. Dieser Bereich stellt also das Maximum der Molekulargewichtsverteilung dar (es liegt z.B. bei der in der Abbildung aufgeführten Gelatine Typ B bei 95 kD). Der Begriff des "Maximums der Molekulargewichtsverteilung" ist jedoch streng zu trennen von dem Begriff des "durchschnittlichen mittleren Molekulargewichts". Dieser Mittelwert liegt bei der erwähnten Gelatine vom Typ B bei 165 kD.

Solche Gelatinen besitzen in weiten pH-Bereichen eine erhöhte Pufferkapazität und fördern durch ihre hochviskose Eigenschaft die Ausbildung eines physiologischen "Nanomilieus". Sie erhöhen damit die therapeutische Wirkung und Verträglichkeit im Sinne der Erfindung.

Durch Kombination der beschriebenen Vorgehensweise lassen sich Gelatinesorten finden, die technologisch gesehen auf überraschend einfache Weise zu Retardarzneiformen mit neuen Eigenschaften führen.

Die Herstellung der erfindungsgemäßen Matrix bzw. des Nanosols erfolgt analog zu den o.g. Patentanmeldungen (11 AL2713 und 11 AL2703) angeführten Verfahren einschließlich der beschriebenen Vortests, wobei in bevorzugten Ausführungsformen die oben näher bezeichneten Gelatinesorten einzusetzen sind.

Die Nanosole können beispielsweise sprühgetrocknet werden, wobei ein Zusatz von PVP oder anderer Hilfsstoffe aus technologischer Sicht grundsätzlich möglich ist.

Weiterhin können andere synthetische oder natürliche Makromoleküle zugesetzt werden, sofern sie sich nicht störend auf die Resorption auswirken.

Wegen der verschiedenen Wirkungsmechanismen für das S- und das R-Enantiomere (S-Ibuprofen weist in erster Linie periphere Wirkung auf, während das enantiomere R-Ibuprofen aufgrund des spezifischen Metabolismus und der damit zuammenhängenden Gewebeverteilung vor allem zentral wirkt) können Mischungen aus S- und R-Enantiomeren mit verschiedenen Anteilen der einzelnen Enantiomeren im Einzelfall bevorzugt sein.

Folgende Beispiele sollen die vorliegende Erfindung näher erläutern:

**Beispiel 1:**

| | |
|---|---|
| Wirkstoff: | S-Ibuprofen, enantiomerreine Wirkstoffsäure |
| Gelatinetyp: | Typ B (IEP 4,9), 320 Bloom, Mikrogelanteil 17 %, Herstellung Beispiel II |
| Nanosol-Herstellung: | analog Verfahren II |
| Gewichtsverhältnis Gelatine/Wirkstoff: | 1,5 : 1 |

300 g oben genannter Gelatine werden in 5 l Wasser gelöst. 200 g S-Ibuprofen werden in 0,6 l Natronlauge (10%ig) gelöst und zur Gelatinelösung gegeben. Danach wird durch Zugabe von Salzsäure auf pH 3.0 eingestellt, wobei sich das Nanosol bildet.

Teilchengrößenmessungen ergeben zu 80% Teilchengrößen der Nanosolpartikel von kleiner 360 nm.

Das sprühgetrocknete Pulver wird zu Retardtabletten mit einer Dosierung von 200 mg S-Ibuprofen auf einer Exzenterpresse verpreßt. Die Tabletten werden nicht magensaftresistent überzogen.

**Beispiel 2:**

| | |
|---|---|
| Wirkstoff: | S-Ibuprofen, enantiomerreine Wirkstoffsäure |
| Gelatinetyp: | wie in Beispiel 1, jedoch vollentsalzt |
| Nanosol-Herstellung: | analog Verfahren III |
| Gewichtsverhältnis Gelatine/Wirkstoff: | 3 : 1 |

600 g der oben genannten Gelatine werden in 10 l destilliertem Wasser bei 50 °C gelöst und auf einen pH-Wert von 3,0 eingestellt.

200 g S-Ibuprofen werden in 0,5 l Ethanol gelöst. Die alkoholische Lösung wird mit der Gelatinelösung bei 40 °C homogen vermischt, das organische Lösungsmittel wird bei gleichzeitiger Bildung des Nanosols unter Vakuum entfernt.

Teilchengrößenmessung der Nanosolpartikel ergeben durchschnittliche Teilchengrößen von 250 nm.

Die erhaltene Lösung wird sprühgetrocknet und das Pulver nach Trockengranulierung zu Matrixtabletten mit einem Gehalt von 200 mg S-Ibuprofen auf einer Exzenterpresse komprimiert.

**Beispiel 3:**

Die in Beispiel 2 hergestellten Tabletten werden in einem Dissolutiontest nach USP (s. Fig.1) bei unterschiedlichen pH-Werten gegenüber herkömmlich magensaftresistent überzogenen Filmtabletten gemessen (0-120 Min. Magensaft pH 1,2; 120-550 Min. Darmsaft pH 6,5). Die pH-unabhängigen Freigabe der Nanosol-Tablette erfolgt kontinuierlich innerhalb von 8 Stunden zu 100%.

**Beispiel 4:**

In Fig. 2 wird der Plasmakonzentrations-Zeitverlauf der in Beispiel 2 hergestellten Tablettencharge im Vergleich zu einer herkömmlichen Retardzubereitung dargestellt. Die Nanosoltablette zeigt eine über mehrere Stunden anhaltende steady-state-Phase mit einem, bedingt durch sofortige Resorption im Magen, deutlich früheren Wirkeintritt.

## Patentansprüche

**1.** Retard-Arzneimittel zur Behandlung von schmerzhaften und/oder endzündlichen sowie fieberhaften Erkrankungen, enthaltend Ibuprofen neben üblichen pharmazeutischen Trägern und Hilfsstoffen, dadurch gekennzeichnet, daß das Ibuprofen in Form eines pharmazeutisch applizierbaren Nanosols vorliegt, das als Träger im wesentlichen Gelatine, fraktionierte Gelatine oder ein Gelatinederivat enthält.

**2.** Arzneimittel nach Anspruch 1, dadurch gekennzeichnet daß das Nanosol
a) eine innere Phase aus dem Ibuprofen, das eine Teilchengröße von 10 - 800 nm aufweist und eine Oberflächenladung besitzt,
b) eine äußere Phase aus Gelatine, fraktionierter Gelatine oder einem Gelatinederivat, welche(s) gegensinnig geladen ist, und
c) einen annähernden oder vollständigen isoionischen Ladungszustand der inneren und äußeren Phase aufweist, und
d) physiologisch resorbierbar ist.

**3.** Arzneimittel nach Anspruch 1 und/oder 2, dadurch gekennzeichnet, daß das Ibuprofen als feste, resuspendierbare Nanodispersion vorliegt.

**4.** Arzneimittel nach einem der Ansprüche 1 bis 3, dadurch gekennzeichnet, daß das Ibuprofen eine durchschnittliche Teilchengröße unterhalb von 400 nm aufweist.

**5.** Arzneimittel nach einem der Ansprüche 1 - 4, dadurch gekennzeichnet, daß die Gelatine ein Maximum der Molekulargewichtsverteilung im Bereich von $10^4$ bis $10^7$ D aufweist.

**6.** Arzneimittel nach Anspruch 5, dadurch gekennzeichnet, daß die Gelatine ein Maximum der Molekulargewichtsverteilung oberhalb von $9{,}5 \times 10^4$ D und einen Peptidanteil kleiner als 5 Gewichtsprozent aufweist.

**7.** Arzneimittel nach einem der Ansprüche 1 - 6, dadurch gekennzeichnet, daß die Gelatine einen prozentualen Gewichtsanteil der Mikrogelfraktion ($> 10^7$ D) größer als 15 % Prozent aufweist.

**8.** Arzneimittel nach einem der Ansprüche 1 bis 7, gekennzeichnet durch eine äußere Phase des Nanosols, die zusätzlich viskositätserhöhende Stoffe in einem Gewichtsverhältnis von Gelatine zu synthetischem oder natürlichem Polymer wie 10:1 bis 1000:1 enthält.

**9.** Arzneimittel nach einem der Ansprüche 1 - 8, dadurch gekennzeichnet, daß die Retardform eine Tablette ist.

**10.** Arzneimittel nach Anspruch 9, dadurch gekennzeichnet, daß die Retardform eine Matrixtablette ist.

**11.** Arzneimittel zur Behandlung von schmerzhaften und/oder entzündlichen sowie fieberhaften Erkrankungen, enthaltend Ibuprofen neben üblichen pharmazeutischen Trägern und Hilfsstoffen, dadurch gekennzeichnet, daß es teilweise als Akutform, teilweise als Retardform mit Ibuprofen in Form eines pharmazeutisch applizierbaren Nanosols vorliegt.

**12.** Arzneimittel nach Anspruch 11, dadurch gekennzeichnet, daß die Akut- und Retardform in einer Hartgelatinekapsel vorliegen.

**13.** Arzneimittel nach einem der Ansprüche 1-12, dadurch gekennzeichnet, daß das Ibuprofen als Racemat vorliegt.

**14.** Arzneimittel nach einem der Ansprüche 1-12, dadurch gekennzeichnet, daß das Ibuprofen als Pseudoracemat vorliegt.

**15.** Arzneimittel nach einem der Ansprüche 1-12, dadurch gekennzeichnet, daß das Ibuprofen als reines S- oder R-Ibuprofen vorliegt.

**16.** Arzneimittel nach einem der Ansprüche 1-12, dadurch gekennzeichnet, daß das Ibuprofen als Gemisch von S- und R-Enantiomeren vorliegt.

17. Arzneimittel nach Anspruch 16, dadurch gekennzeichnet, daß das Ibuprofen zu mindestens 50 % als R-Ibuprofen vorliegt.

18. Arzneimittel nach Anspruch 16, dadurch gekennzeichnet, daß das Ibuprofen zu mindestens 50 % als S-Ibuprofen vorliegt.

19. Verwendung eines pharmazeutisch applizierbaren Nanosols von Ibuprofen, das zu mindestens 50% als S- oder R-Ibuprofen vorliegt, zur Herstellung von Arzneimitteln mit analgetischer und/oder antirheumatischer Retardwirkung.

**Claims**

1. Slow-release medicament for the treatment of painful and/or inflammatory and febrile disorders, containing ibuprofen in addition to customary pharmaceutical excipients and auxiliaries, characterized in that the ibuprofen is present in the form of a pharmaceutically administrable nanosol which, as an excipient, essentially contains gelatin, fractionated gelatin or a gelatin derivative.

2. Medicament according to Claim 1, characterized in that the nanosol
   a) contains an inner phase comprising the ibuprofen, which has a particle size of 10 - 800 nm and possesses a surface charge,
   b) an outer phase of gelatin, fractionated gelatin or a gelatin derivative, which is oppositely charged, and
   c) has a nearly or completely isoionic state of charge of the inner and outer phase, and
   d) is physiologically absorbable.

3. Medicament according to Claim 1 and/or 2, characterized in that the ibuprofen is present as a solid, resuspendable nanodispersion.

4. Medicament according to one of Claims 1 to 3, characterized in that the ibuprofen has an average particle size of below 400 nm.

5. Medicament according to one of Claims 1 - 4, characterized in that the gelatin has a maximum of the molecular weight distribution in the range from $10^4$ to $10^7$ D.

6. Medicament according to Claim 5, characterized in that the gelatin has a maximum of the molecular weight distribution of above $9.5 \times 10^4$ D and a peptide content of less than 5 per cent by weight.

7. Medicament according to one of Claims 1 - 6, characterized in that the gelatin has a percentage weight content of the microgel fraction ($> 10^7$ D) of greater than 15%.

8. Medicament according to one of Claims 1 - 7, characterized by an outer phase of the nanosol which additionally contains viscosity-increasing substances in a weight ratio of gelatin to synthetic or natural polymer much as 10:1 to 1000:1.

9. Medicament according to one of Claims 1 - 8, characterized in that the slow-release form is a tablet.

10. Medicament according to Claim 9, characterized in that the slow-release form is a matrix tablet.

11. Medicament for the treatment of painful and/or inflammatory and febrile disorders, containing ibuprofen in addition to customary pharmaceutical excipients and auxiliaries, characterized in that it is partially present as an immediate-effect form and partially as a slow-release form containing ibuprofen in the form of a pharmaceutically administrable nanosol.

12. Medicament according to Claim 11, characterized in that the immediate-effect and slow-release forms are present in a hard gelatin capsule.

13. Medicament according to one of Claims 1 - 12, characterized in that the ibuprofen is present as a racemate.

14. Medicament according to one of Claims 1 - 12, characterized in that the ibuprofen is present as a pseudoracemate.

15. Medicament according to one of Claims 1 - 12, characterized in that the ibuprofen is present as pure S- or R-ibuprofen.

16. Medicament according to one of Claims 1 - 12, characterized in that the ibuprofen is present as a mixture of S- and R-enantiomers.

17. Medicament according to Claim 16, characterized in that the ibuprofen is present to at least 50% as R-ibuprofen.

18. Medicament according to Claim 16, characterized in that the ibuprofen is present to at least 50% as S-ibuprofen.

19. Use of a pharmaceutically administrable nanosol of ibuprofen which is present to at least 50% as S- or R-ibuprofen for the production of medicaments having analgesic and/or antirheumatic slow-release action.

**Revendications**

1. Médicament à action retardée destiné au traitement de maladies accompagnées de douleur et/ou d'inflammation ainsi que de fièvre, contenant de l'ibuprofène à côté de supports et de substances auxiliaires pharmaceutiques classiques, caractérisé en ce que l'ibuprofène est présent sous forme d'un nanosol pharmaceutiquement acceptable, qui contient comme support principalement de la gélatine, de la gélatine fractionnée ou un dérivé de gélatine.

2. Médicament suivant la revendication 1, caractérisé en ce que le nanosol présente
   a) une phase interne formée de l'ibuprofène qui présente des diamètres de particules de 10 à 800 nm et qui possède une charge de surface,
   b) une phase externe formée de gélatine, de gélatine fractionnée ou d'un dérivé de gélatine, qui porte une charge opposée et
   c) un état de charge presque isoionique ou tout à fait isoionique des phases interne et externe, et
   d) peut être résorbé physiologiquement.

3. Médicament suivant la revendication 1 et/ou la revendication 2, caractérisé en ce que l'ibuprofène se présente comme nano-dispersion solide pouvant être remise en suspension.

4. Médicament suivant l'une des revendications 1 à 3, caractérisé en ce que l'ibuprofène présente un diamètre moyen de particules inférieur à 400 nm.

5. Médicament suivant l'une des revendications 1 à 4, caractérisé en ce que la gélatine présente un maximum de répartition de poids moléculaire dans la plage de $10^4$ à $10^7$ D.

6. Médicament suivant la revendication 5, caractérisé en ce que la gélatine présente un maximum de distribution de poids moléculaire au-dessus de $9{,}5 \times 10^4$ D et une proportion de peptide inférieure à 5 % en poids.

7. Médicament suivant l'une des revendications 1 à 6, caractérisé en ce que la gélatine présente un pourcentage en poids de la fraction de microgel (> $10^7$ D) supérieure à 15 %.

8. Médicament suivant l'une des revendications 1 à 7, caractérisé par une phase externe du nanosol qui contient en outre des substances élevant la viscosité dans un rapport en poids de la gélatine au polymère synthétique ou naturel de 10:1 à 1000:1.

9. Médicament suivant l'une des revendications 1 à 8, caractérisé en ce que la forme à action retardée est un comprimé.

**10.** Médicament suivant la revendication 9, caractérisé en ce que la forme à action retardée est un comprimé à matrice.

**11.** Médicament destiné au traitement de maladies accompagnées de douleur et/ou d'inflammation ainsi que de fièvre, contenant de l'ibuprofène, à côté de supports et de substances auxiliaires pharmaceutiques classiques, caractérisé en ce qu'il se présente en partie sous une forme à action rapide et en partie sous une forme à action retardée avec de l'ibuprofène sous forme d'un nanosol pharmaceutiquement applicable.

**12.** Médicament suivant la revendication 11, caractérisé en ce que la forme à action rapide et la forme à action retardée sont présentes dans une capsule de gélatine dure.

**13.** Médicament suivant l'une des revendications 1 à 12, caractérisé en ce que l'ibuprofène se présente comme racémate.

**14.** Médicament suivant l'une des revendications 1 à 12, caractérisé en ce que l'ibuprofène se présente comme pseudo-racémate.

**15.** Médicament suivant l'une des revendications 1 à 12, caractérisé en ce que l'ibuprofène se présente comme S- ou R-ibuprofène pur.

**16.** Médicament suivant l'une des revendications 1 à 12, caractérisé en ce que l'ibuprofène se présente comme mélange de S- et R-énantiomères.

**17.** Médicament suivant la revendication 16, caractérisé en ce que l'ibuprofène se présente en proportion d'au moins 50 % sous forme de R-ibuprofène.

**18.** Médicament suivant la revendication 16, caractérisé en ce que l'ibuprofène se présente en proportion d'au moins 50 % sous forme de S-ibuprofène.

**19.** Utilisation d'un nanosol d'ibuprofène pharmaceutiquement applicable, qui se présente en proportion d'au moins 50 % comme S- ou R-ibuprofène, pour la préparation de médicaments à action analgésique et/ou anti-rhumatismale retardée.

Fig. 1

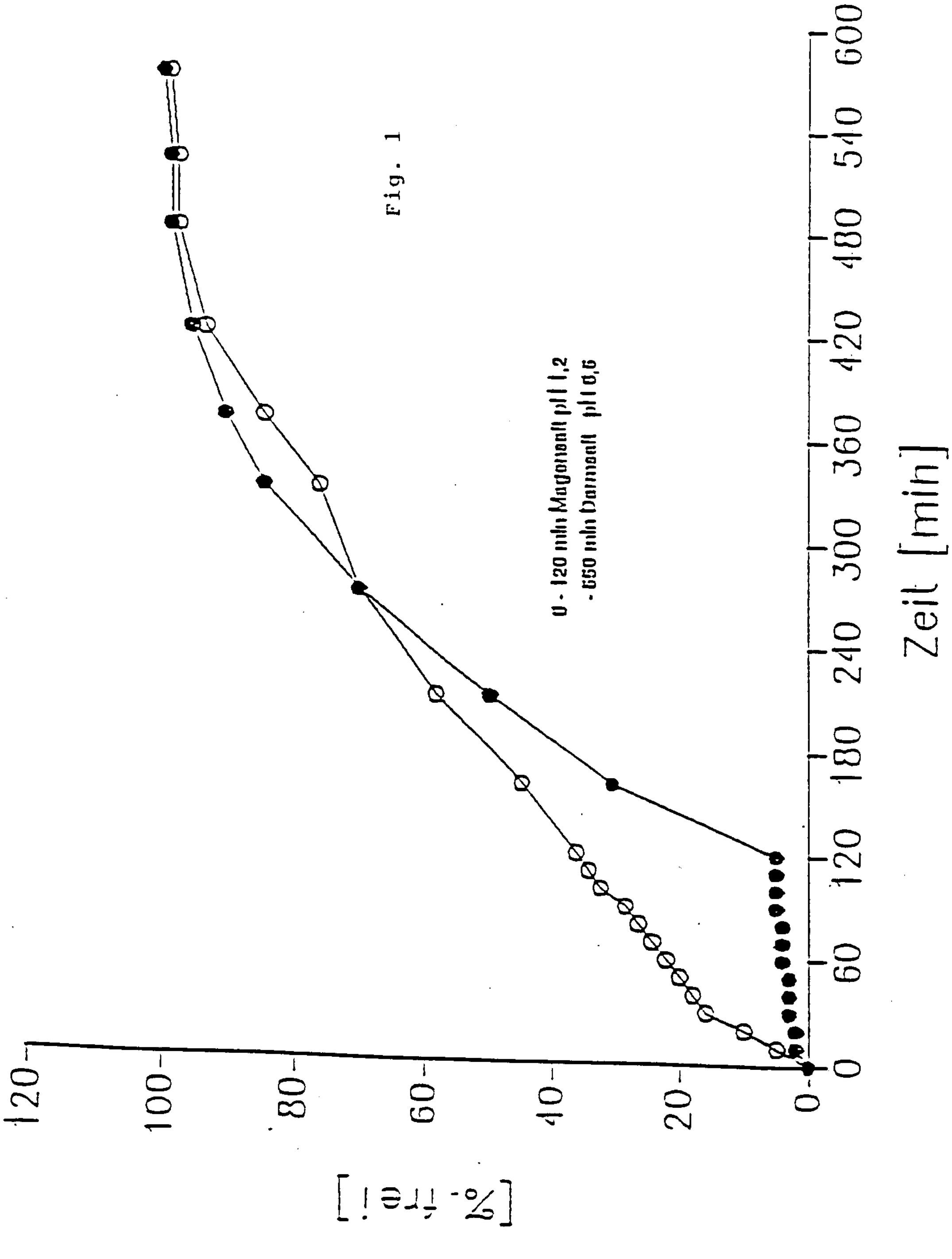


Auflösungsprofil von S-Ibuprofen aus 200 mg Retardzubereitungen (• handelsübliche Filmtablette; o Nanosol Tablette aus Beispiel)

Fig. 2

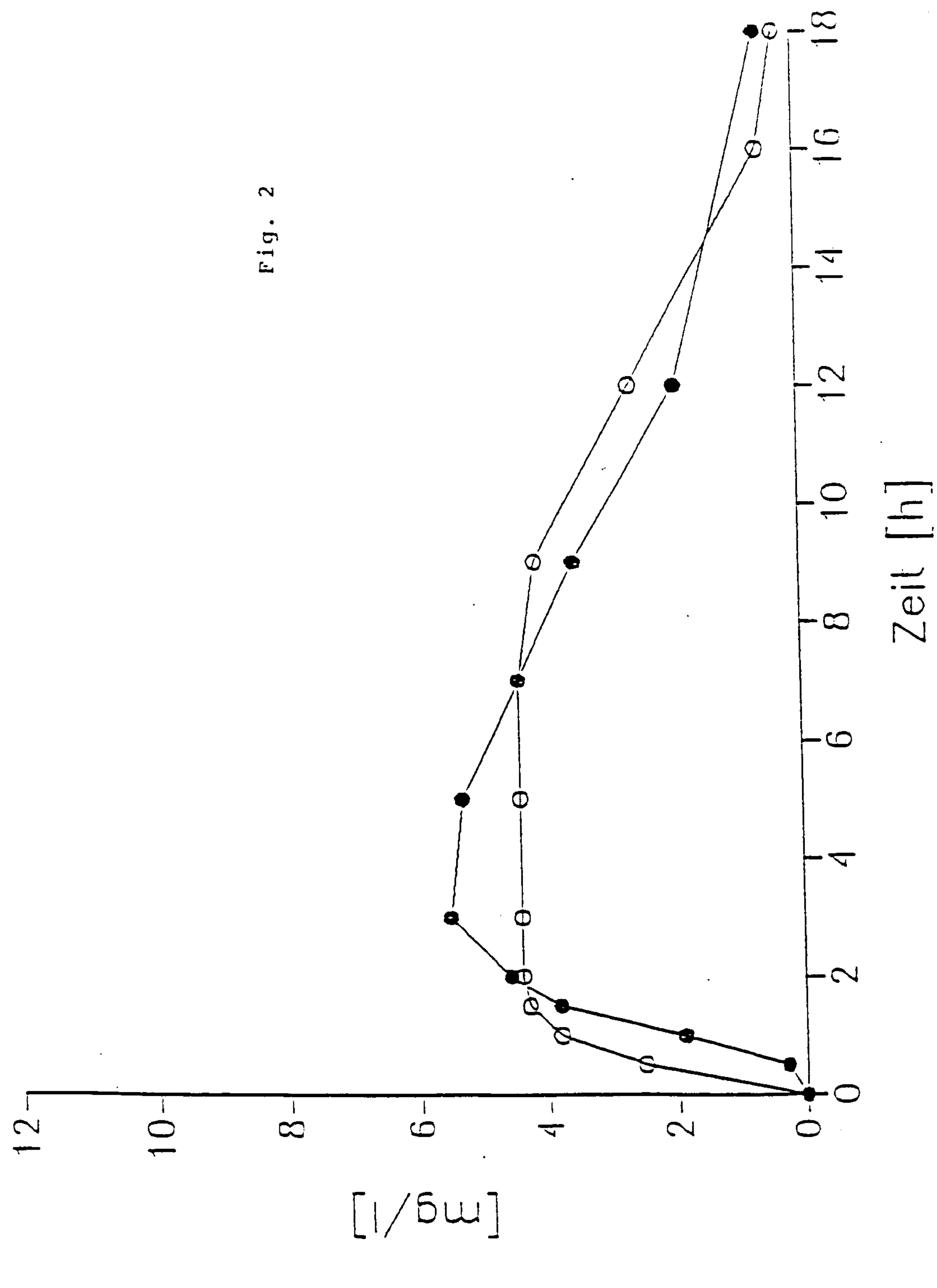


Plasmakonzentrations-Zeitverlauf von S-Ibuprofen nach oraler Gabe von 200 mg als herkömmliche Retardtablette (•) bzw. als Nanosol Retardtablette (o)

Fig. 3

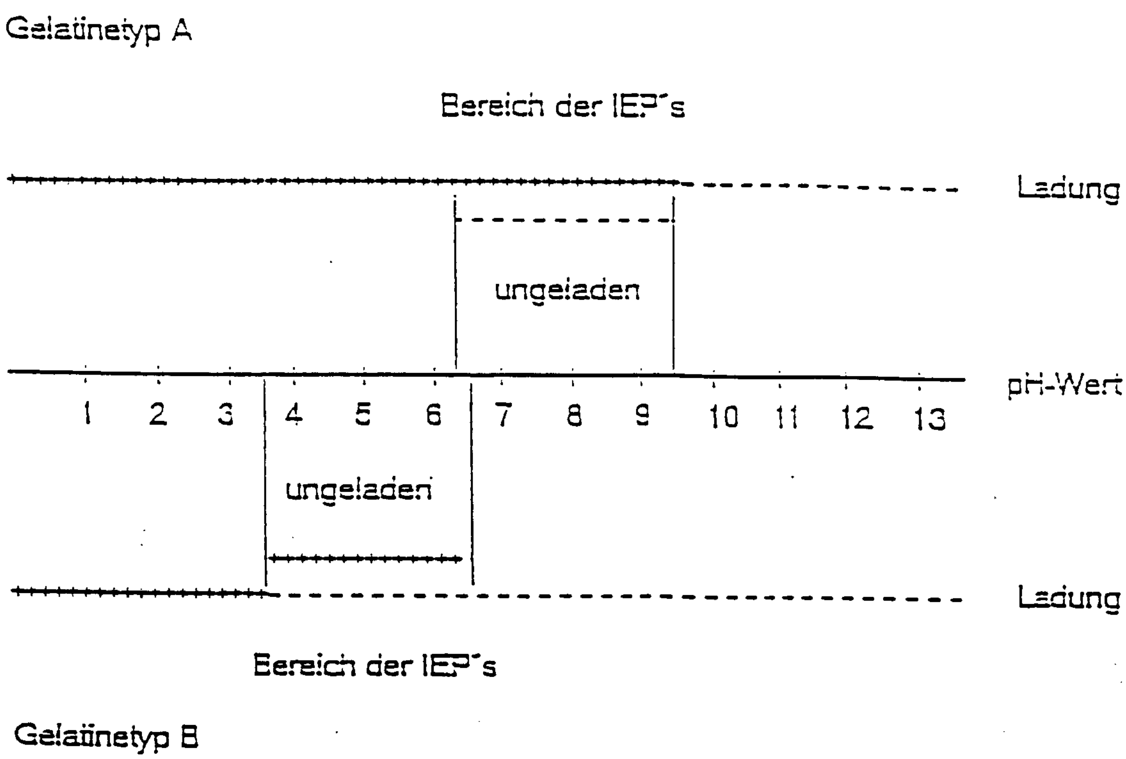


Ladungsverteilungen in den Gelatinetypen A (sauer) und B (alkalisch)

IEP = isoelektrischer Punkt

Fig. 4

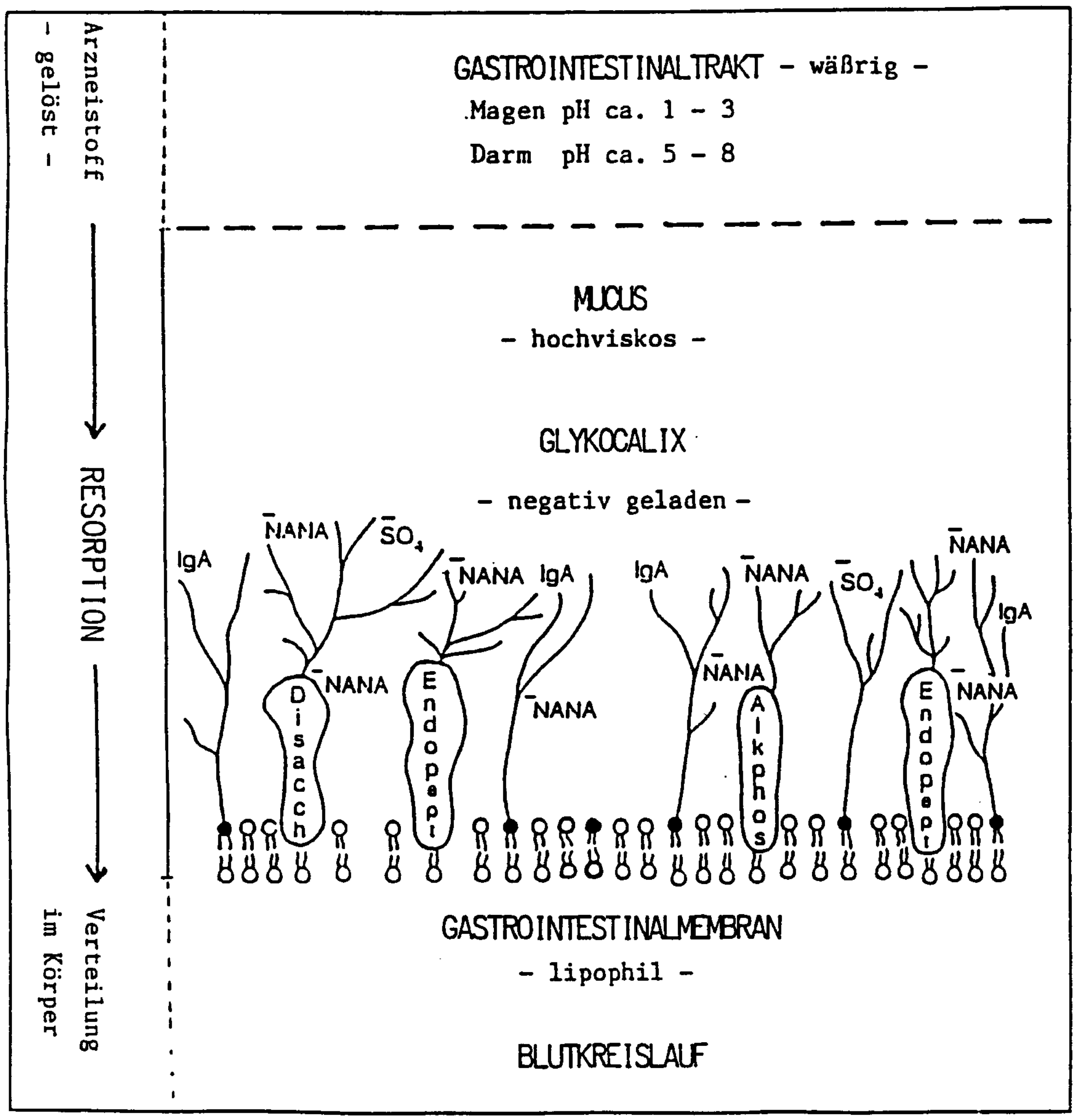
Arzneistoff
- gelöst -
RESORPTION
Verteilung
im Körper
GASTROINTESTINALTRAKT - wäßrig -
Magen pH ca. 1 - 3
Darm pH ca. 5 - 8
MUCUS
- hochviskos -
GLYKOCALIX
- negativ geladen -
IgA
NANA
SO4
Disacch
Endopept
Alkphos
GASTROINTESTINALMEMBRAN
- lipophil -
BLUTKREISLAUF